# EUROPEAN PATENT APPLICATION

(11) **EP 4 471 786 A1**
(43) Date of publication of application: **04.12.2024**
(21) Application number: 22923419.0
(22) Date of filing: 18.11.2022
(51) Int. Cl.: G16B 40/00

(54) **METHYLATION SEQUENCING DATA FILTERING METHOD AND APPLICATION**

(30) Priority: 27.01.2022 CN 202210101807
(71) Applicant: Ankang Euler Technology Limited Company, Ankang, Shaanxi 725029 (CN)
(72) Inventor: ZHANG, Yi, Ankang, Shaanxi 725029 (CN); CHEN, Xintong, Ankang, Shaanxi 725029 (CN)
(74) Representative: Bardehle Pagenberg Partnerschaft mbB Patentanwälte Rechtsanwälte
(86) International application number: PCT/CN2022/132767
(87) International publication number: WO 2023/142625

(57) **Abstract**

The present invention relates to the technical field of biology, and in particular to a methylation sequencing data filtering method and an application. According to the methylation sequencing data filtering method provided by the present invention, the efficiency of converting an unmethylated C base into a U base in DNA treated with bisulfite can be improved from 98.81% to 99.44%. On this basis, by applying the data filtering method provided by the present application, the methylation level of a sequence can be accurately evaluated, and a methylated haplotype having high quality, high accuracy and high credibility can be obtained. Experimental data shows that by using the methylated haplotype obtained by means of the methylation sequencing data filtering method provided by the present invention, the urine sample of a urothelial carcinoma patient can be well distinguished from that of a normal person (AUC=0.94), and the urine sample of a patient with muscle-invasive urothelial carcinoma can be well distinguished from that of a patient with non-muscle-invasive urothelial carcinoma (AUC=0.967).

## Description

### CROSS-REFERENCE TO RELATED APPLICATION

The present application claims the priority to and the benefit of Chinese Patent Application No. 202210101807.9 filed with the Patent Office of CNIPA on January 27, 2022 and entitled "METHYLATION SEQUENCING DATA FILTERING METHOD AND APPLICATION", the entire contents of which are incorporated herein by reference.

### TECHNICAL FIELD

The present disclosure pertains to the field of biotechnology, and specifically relates to a methylation sequencing data filtering method and an application.

### BACKGROUND

DNA methylation is an important epigenetic modification and related to many biological regulatory pathways. There are currently a variety of methylation quantitative methods with a base-level resolution. Bisulfite sequencing (BS-seq or methseq) is adopted in the present disclosure.

In the bisulfite-treated DNA, the unmethylated C base is converted into the U base and the conversion efficiency can reach about 99%. Methylation modifications at the single-base level may be accurately measured by the high-throughput sequencing method. After the bisulfite-converted DNA is amplified by the PCR reaction, the U base is converted into the T base, while the methylated C base remains unchanged, so the specific base position at which the methylation modification occurs can be determined from the base information at each position of the sequence. The computer software determines the bases and their sequential order measured by the sequencer, obtains the sequence reads/fragments, and outputs the data in FASTQ format recording the read sequence and sequencing quality scores.

In conventional analysis, the following two errors may possibly occur to the bisulfite sequencing data: (1) the unmethylated C base has not been successfully converted into the T base after treatment; and (2) the methylated C base that should have remained unchanged has changed. However, there is currently no better experimental means to avoid the errors occurring in the process of DNA treatment with bisulfite.

### SUMMARY

### Problems to be solved

The present disclosure provides a methylation sequencing data filtering method for improving the efficiency of converting the unmethylated C bases into the U bases when treating DNA with bisulfite. Also, the present disclosure provides an application of the methylation sequencing data filtering method in cancer detection to create a cancer prediction model with high sensitivity and good specificity.

### Solution to the problems

[1]. An apparatus for filtering methylation sequencing data, comprising:
   a processor; and
   a memory configured to store executable instructions;
   wherein the processor, when executing instructions, implements a method for filtering the methylation sequencing data, the method comprising the following steps:
      i) truncating a sequencing sequence on an insertion region during primer synthesis;
      ii) filtering a sequencing read according to the following conditions to obtain a methylation haplotype of the sequencing read that satisfies the conditions:
         a. removing a read with a C base and a G base both having a conversion frequency of less than 0.9; b. removing a read with a C base and a G base both having a conversion frequency of greater than 0.5; c. removing a sequence deemed to be erroneous; d. retaining a sequencing read with a conversion frequency of a C base less than 0.2 while a conversion frequency of a G base greater than 0.8; and e. retaining a sequencing read with a conversion frequency of a G base less than 0.2 while a conversion frequency of a C base greater than 0.8;
      iii) extracting a haplotype with a sequencing depth greater than 10; and
      iiii) filtering out a haplotype with a methylation frequency of a C base being null.
[2]. The apparatus according to [1], wherein the methylation sequencing data is obtained by bisulfite sequencing.
[3]. An apparatus for selecting a DNA methylation haplotype capable of determining whether a subject suffers from cancer or determining subtypes of cancer in a patient, comprising:
   a processor; and
   a memory configured to store executable instructions;
   wherein the processor, when executing instructions, implements a method for selecting the DNA methylation haplotype capable of determining whether a subject suffers from cancer or determining subtypes of cancer in a patient, the method comprising the following steps:
      a step of filtering methylation sequencing data;
      a step of selecting differential methylation haplotypes; and
      a step of training the differential methylation haplotypes.
[4]. The apparatus according to [3], wherein the step of filtering methylation sequencing data comprises:
   i) truncating a sequencing sequence on an insertion region during primer synthesis;
   ii) filtering a sequencing read according to the following conditions to obtain a methylation haplotype of the sequencing read that satisfies the conditions: a. removing a read with a C base and a G base both having a conversion frequency of less than 0.9; b. removing a read with a C base and a G base both having a conversion frequency of greater than 0.5; c. removing a sequence deemed to be erroneous; d. retaining a sequencing read with a conversion frequency of a C base less than 0.2 while a conversion frequency of a G base greater than 0.8; and e. retaining a sequencing read with a conversion frequency of a G base less than 0.2 while a conversion frequency of a C base greater than 0.8;
   iii) extracting a haplotype with a sequencing depth greater than 10; and
   iiii) filtering out a haplotype with a methylation frequency of a C base being null.
[5]. The apparatus according to [3], wherein the step of selecting differential methylation haplotypes comprises:
   performing a Fisher's test on the methylation haplotypes that have completed the step of filtering methylation sequencing data, and selecting differential methylation haplotypes specific to different subtypes of cancer cells that satisfy the following conditions:
   a significance indicated as p of pairwise difference between the different subtypes of cancer cells and normal leukocytes is less than 0.05, a difference comparison of the methylation haplotype is performed in a sample at a cancer cell DNA concentration of 1, each of the ratios among methylation levels of the different subtypes of cancer cells is required to be greater than 2 or less than 1/2, and each of the ratios between methylation levels of the different subtypes of cancer cells and that of a normal leukocyte sample is additionally required to be greater than 2 or less than 1/2; the differential methylation haplotype specific to leukocytes that satisfies the following condition is selected: multiples of differences between methylation level of a leukocyte sample and those of the different subtypes of cancer samples are greater than 3, and a haplotype frequency in the cancer samples is not less than 0.0001;
   creating the following linear models and obtaining a minimum detection limit of a cancer cell DNA content:
      i) the linear models with the respective sums of an abundance of the differential methylation haplotype specific to a normal human leukocyte and an abundance of each of the differential methylation haplotypes specific to the different subtypes of cancer cells varying with a cancer cell DNA concertation; and
      ii) the linear model with an abundance of the differential methylation haplotype specific to a normal human leukocyte varying with a normal leukocyte DNA concentration.
[6]. The apparatus according to [3], wherein the step of training the differential methylation haplotypes comprises:
   detecting differences between methylation levels of different subtypes of cancer tissue samples and that of a normal urine sample and the difference among the methylation levels of the different subtypes of cancer samples, selecting a haplotype with a significance indicated as p of difference less than 0.05 in three t-test results, and categorizing the haplotype depending upon whether a logarithm of a mean difference value of the methylation levels is positive or negative, and accordingly making a determination in a tissue sample and a urine sample respectively, and calculating sensitivity and specificity of a selected differential methylation haplotype for distinguishing the cancer samples from a normal human sample and for determining the different subtypes of the cancer samples.
[7]. The apparatus according to any one of [3] to [6], wherein the methylation sequencing data is obtained by bisulfite sequencing.
[8]. The apparatus according to any one of [3] to [6], wherein the sample is gDNA or cfDNA.
[9]. The apparatus according to any one of [3] to [6], wherein the cancer is bladder urothelial carcinoma.
[10]. The apparatus according to any one of [3] to [6], wherein the subtypes of cancer are muscle invasive bladder urothelial carcinoma and non-muscle invasive bladder urothelial carcinoma.
[11]. A method for filtering methylation sequencing data, comprising the following steps:
   i) truncating a sequencing sequence on an insertion region during primer synthesis;
   ii) filtering a sequencing read according to the following conditions to obtain a methylation haplotype of the sequencing read that satisfies the conditions:
      a. removing a read with a C base and a G base both having a conversion frequency of less than 0.9; b. removing a read with a C base and a G base both having a conversion frequency of greater than 0.5; c. removing a sequence deemed to be erroneous; d. retaining a sequencing read with a conversion frequency of a C base less than 0.2 while a conversion frequency of a G base greater than 0.8; and e. retaining a sequencing read with a conversion frequency of a G base less than 0.2 while a conversion frequency of a C base greater than 0.8;
   iii) extracting a haplotype with a sequencing depth greater than 10; and
   iiii) filtering out a haplotype with a methylation frequency of a C base being null.
[12]. The method according to [11], wherein the methylation sequencing data is obtained by bisulfite sequencing.
[13]. A method for selecting a DNA methylation haplotype capable of determining whether a subject suffers from cancer or determining subtypes of cancer in a patient, comprising the following steps:
   a step of filtering methylation sequencing data;
   a step of selecting differential methylation haplotypes; and
   a step of training the differential methylation haplotypes.
[14]. The method according to [13], wherein the step of filtering methylation sequencing data comprises:
   i) truncating a sequencing sequence on an insertion region during primer synthesis;
   ii) filtering a sequencing read according to the following conditions to obtain a methylation haplotype of the sequencing read that satisfies the conditions: a. removing a read with a C base and a G base both having a conversion frequency of less than 0.9; b. removing a read with a C base and a G base both having a conversion frequency of greater than 0.5; c. removing a sequence deemed to be erroneous; d. retaining a sequencing read with a conversion frequency of a C base less than 0.2 while a conversion frequency of a G base greater than 0.8; and e. retaining a sequencing read with a conversion frequency of a G base less than 0.2 while a conversion frequency of a C base greater than 0.8;
   iii) extracting a haplotype with a sequencing depth greater than 10; and
   iiii) filtering out a haplotype with a methylation frequency of a C base being null.
[15]. The method according to [13], wherein the step of selecting differential methylation haplotypes comprises:
   performing a Fisher's test on the methylation haplotypes that have completed the step of filtering methylation sequencing data, and selecting differential methylation haplotypes specific to different subtypes of cancer cells that satisfy the following conditions:
   a significance indicated as p of pairwise difference between the different subtypes of cancer cells and normal leukocytes is less than 0.05, a difference comparison of the methylation haplotype is performed in a sample at a cancer cell DNA concentration of 1, each of the ratios among methylation levels of the different subtypes of cancer cells is required to be greater than 2 or less than 1/2, and each of the ratios between methylation levels of the different subtypes of cancer cells and that of a normal leukocyte sample is additionally required to be greater than 2 or less than 1/2; the differential methylation haplotype specific to leukocytes that satisfies the following condition is selected: multiples of differences between methylation level of a leukocyte sample and those of the different subtypes of cancer samples are greater than 3, and a haplotype frequency in the cancer samples is not less than 0.0001;
   creating the following linear models and obtaining a minimum detection limit of a cancer cell DNA content:
      i) the linear models with the respective sums of an abundance of the differential methylation haplotype specific to a normal human leukocyte and an abundance of each of the differential methylation haplotypes specific to the different subtypes of cancer cells varying with a cancer cell DNA concertation; and
      ii) the linear model with an abundance of the differential methylation haplotype specific to a normal human leukocyte varying with a normal leukocyte DNA concentration.
[16]. The method according to [13], wherein the step of training the differential methylation haplotypes comprises:
   detecting differences between methylation levels of different subtypes of cancer tissue samples and that of a normal urine sample and the difference among the methylation levels of the different subtypes of cancer samples, selecting a haplotype with a significance indicated as p of difference less than 0.05 in three t-test results, and categorizing the haplotype depending upon whether a logarithm of a mean difference value of the methylation levels is positive or negative, and accordingly making a determination in a tissue sample and a urine sample respectively, and calculating sensitivity and specificity of a selected differential methylation haplotype for distinguishing the cancer samples from a normal human sample and for determining the different subtypes of the cancer samples.
[17]. The method according to any one of [13] to [16], wherein the methylation sequencing data is obtained by bisulfite sequencing.
[18]. The method according to any one of [13] to [16], wherein the sample is gDNA or cfDNA.
[19]. The method according to any one of [13] to [16], wherein the cancer is bladder urothelial carcinoma.
[20]. The method according to any one of [13] to [16], wherein the subtypes of cancer are muscle invasive bladder urothelial carcinoma and non-muscle invasive bladder urothelial carcinoma.

### Advantageous effects

By implementing the above technical solutions, the methylation sequencing data filtering method provided in the present disclosure can increase the efficiency of converting the unmethylated C bases in bisulfite-treated DNA into U bases from 98.81% to 99.44%. Furthermore, the data filtering method of the present disclosure can be applied to correctly assess the methylation level of the sequence and obtain methylation haplotypes with reliable quality and high accuracy and credibility. The experimental data show that the methylation haplotypes obtained by the methylation sequencing data filtering method provided herein can be used to well distinguish the urine sample of a patient with bladder urothelial carcinoma from the urine sample of a normal person (AUC=0.94) and distinguish the urine sample of a patient with muscle invasive bladder urothelial carcinoma from the urine sample of a patient with non-muscle invasive bladder urothelial carcinoma (AUC=0.967).

### BRIEF DESCRIPTION OF THE DRAWINGS

FIG. 1 shows a flowchart of methylation sequencing and analysis.
FIG. 2 shows a comparison diagram for the improved effect in methylation conversion rate.
FIG. 3 shows a flowchart of extracting a methylation haplotype.
FIG. 4 shows a flowchart of the linear model prediction for a gradient biological standard.
FIG. 5 shows the linear model prediction for determining the lowest detection limits of the MIBC model, the NMIBC model, and the leukocyte-specific model.
FIG. 6 shows a flowchart of authentic sample model prediction.
FIG. 7 shows results of authentic sample model prediction.

### DETAILED DESCRIPTION

The embodiments of the present disclosure will be illustrated below, but the present disclosure is not limited thereto. The present disclosure is not limited to the configurations described below, and various modifications may be made within the scope as claimed in the invention, while the embodiments or examples resulting from appropriate combination of the technical means disclosed in respective different embodiments or examples are also encompassed in the technical scope of the present disclosure.

Unless otherwise defined, the technical and scientific terms used herein have the same meanings as typically understood by one of ordinary skill in the art to which the present disclosure belongs.

In the present disclosure, the term "a" or "an" or "the" may refer to "one", or refer to "one or more", "at least one", and "one or more than one".

In the present disclosure, the term "including", "having", "comprising" or "containing" is intended to be inclusive or open-ended, and does not exclude additional or unrecited elements or methods and steps.

In the present disclosure, the term "about" means that a value includes the standard deviation of the error caused by the apparatus or method used to measure the value.

While it is applicable to the content disclosed herein that the term "or" is defined only as alternatives and "and/or", the term "or" used herein refers to "and/or" unless otherwise expressly stated to be only alternatives or mutual exclusion between alternatives.

In the present disclosure, "cfDNA" refers to Cell-Free Circulating DNA and is the partially degraded endogenous DNA that is free from extracellular parts in human circulating blood, urine, and other body fluids. cfDNAs are mostly 200 bp or less in length.

In the present disclosure, "gDNA" is "genomic DNA" and refers to the total DNA content of an organism in the haploid state.

In the present disclosure, "bisulfite sequencing" is a technique for detecting methylation on all chromosomal DNAs in cells or tissue. Based on the fact that bisulfite enables the unmethylated cytosine (C) in DNA to be deaminated and converted into uracil (U) while keeping the methylated cytosine unchanged, if a desired fragment is amplified by the PCR, all uracil (U) will be converted into thymine (T), and then the PCR product is subjected to the high-throughput sequencing. By analyzing the methylation differences among different samples, the regulation of the gene expression by the DNA methylation level may be studied. The methylation detection method based on the principle of bisulfite conversion has become a conventional technique for detecting the DNA methylation state and is also well-known to a person skilled in the art. In some specific embodiments, the present disclosure uses the EZ DNA Methylation-Gold bisulfite conversion Kit from the Zymo Research Corporation as a reagent for detecting the DNA methylation state to perform the bisulfite conversion on the sample DNA. In the present disclosure, the sequencing may be carried out based on a high-throughput sequencing platform, for example, the sequencing platforms such as Miseq, Hiseq, Nextseq, and Novaseq from the Illumina Corporation, the sequencing platforms such as Ion Torrent or Ion Proton from the Life Technologies Corporation, the sequencing platforms such as PacBio RS II from the Pacific Biosciences System Corporation, and the sequencing platforms such as GridIONX5 from the Oxford Nanopore Technologies Corporation. In some specific embodiments, the present disclosure performs the high-throughput sequencing based on the Novaseq platform from the Illumina Corporation.

In the present disclosure, "DNA methylation" refers to a process of transferring a methyl group from S-adenosyl methionine (SAM) as a methyl donor to a specific base under the catalysis of DNA methyltransferases in an organism. In mammals, methylation mainly takes place at the 5'C-terminal of nucleotide cytosine residues. On human genomes, a large amount of DNA methylations take place on cytosines in CpG dinucleotides, where C is cytosine, G is guanine, and p is a phosphate group. DNA methylation may also take place on cytosine in nucleotide sequences such as CHG and CHH, where H is adenine, cytosine or thymine. DNA methylation may also take place at, e.g., N6-methyladenine, other than cytosines. In addition, DNA methylation may further be in the form of 5-hydroxymethylcytosine, etc.

In the present disclosure, "DNA methylation modification" may be expressed as a single base methylation modification, or may be expressed as a mean of methylation modifications of multiple bases on a consecutive region, or may be expressed as a methylation haplotype, or may also be expressed as a methylation monosomy.

In the present disclosure, "DNA methylation modification degree", "DNA methylation degree", "DNA methylation level", "methylation level", and "haplotype frequency" may be described by the abundance of methylation haplotype.

In the present disclosure, "methylation haplotype", "DNA methylation haplotype", and "haplotype" may be used interchangeably, and refer to a combination of methylation modifications at two or more consecutive C-base sites on a single DNA fragment. Due to the relevance of the DNA methylation modifications on the same chromosome (DNA strand), the methylation modifications to a plurality of adjacent C-base sites may be correlated with one another, forming a certain fixed form. Therefore, the DNA methylation haplotype is an interlocking pattern of DNA methylation modifications on the same chromosome. The methylation haplotype provides more accurate and stable information than the statistical information of a single methylation site, and is a more effective indicator for early detection of cancer and continuous monitoring of its progression.

In the present disclosure, "abundance of methylation haplotype" refers to the proportion of a specific methylation haplotype obtained from statistical analysis of the methylation haplotype information. That is, abundance of methylation haplotype = the number of DNA fragments matching a specific methylation haplotype/ the total number of DNA fragments fully covering the genomic region with this methylation haplotype.

In the present disclosure, "differential methylation haplotype" refers to a haplotype having significantly different abundances of methylation haplotype in different samples.

In the present disclosure, "conversion rate" and "conversion frequency" may be used interchangeably and refer to the proportion of the C bases converted into T bases at non-CpG sites on the positive strand of DNA, and the proportion of the G bases converted into A bases at non-CpG sites on the negative strand of DNA in the bisulfite sequencing process.

In the present disclosure, "insertion sequence during primer synthesis" refers to the sequence of the fragment of interest amplified by the PCR primer.

In the present disclosure, "sequencing depth" refers to the number of DNA fragments fully covering a genomic region with a certain methylation haplotype resulting from the high-throughput sequencing.

In the present disclosure, "methylation frequency of C base" refers to a value obtained by dividing the number of DNA molecules with the C-base site methylation-modified by the total number of DNA molecules containing the C-base site. In the present disclosure, "methylation frequency of the C base being null" means that the methylation frequency of the C base cannot be obtained as a result of an error in calculation or sequence alignment.

In the present disclosure, "sequence deemed to be erroneous" includes, but is not limited to, sequences with an amplification error, a sequencing error, an alignment error, etc.

In the present disclosure, "cancer cell DNA concentration", "tumor DNA content", and the like may be used interchangeably and refer to the proportion of the cancer cell DNA to the sum of the cancer cell DNA and the normal human leukocyte DNA. In some specific embodiments, the cancer cell DNA concentration in the present disclosure may be 100%, 20%, 10%, 5%, 2%, 1%, 0.5% and/or 0%.

In some specific embodiments, the method for filtering the methylation sequencing data according to the present disclosure comprises the following steps: i) truncating a sequencing sequence on an insertion region during primer synthesis; ii) filtering a sequencing read according to the following conditions: a. removing a read with the C base and the G base both having a conversion frequency of less than 0.9, or less than 0.8, or less than 0.7, or less than 0.6 or less than 0.5; b. removing a read with the C base and the G base both having a conversion frequency of greater than 0.5, or greater than 0.6, or greater than 0.7, or greater than 0.8 or greater than 0.9; c. removing a sequence deemed to be erroneous; d. retaining a sequencing read with a conversion frequency of the C base less than 0.2, or less than 0.1 or less than 0.01 while a conversion frequency of the G base greater than 0.8, or greater than 0.9 or greater than 0.99; e. retaining a sequencing read with a conversion frequency of the G base less than 0.2, or less than 0.1 or less than 0.01 while a conversion frequency of the C base greater than 0.8, or greater than 0.9 or greater than 0.99; and f. obtaining a methylation haplotype; iii) extracting a haplotype with a sequencing depth greater than 10; and iiii) filtering out a haplotype with a methylation frequency of the C base being null.

In some specific embodiments, the method for filtering the methylation sequencing data according to the present disclosure comprises the following steps: i) truncating a sequencing sequence on an insertion region during primer synthesis; ii) filtering a sequencing read according to the following conditions: a. removing a read with the C base and the G base both having a conversion frequency of less than 0.9; b. removing a read with the C base and the G base both having a conversion frequency of greater than 0.5; c. removing a sequence deemed to be erroneous; d. retaining a sequencing read with a conversion frequency of the C base less than 0.2 while a conversion frequency of the G base greater than 0.8; e. retaining a sequencing read with a conversion frequency of the G base less than 0.2 while a conversion frequency of the C base greater than 0.8; and f. obtaining a methylation haplotype; iii) extracting a haplotype with a sequencing depth greater than 10; and iiii) filtering out a haplotype with a methylation frequency of the C base being null.

In some preferred embodiments, the method for filtering the methylation sequencing data according to the present disclosure comprises the following steps: i) truncating a sequencing sequence on an insertion region during primer synthesis; ii) filtering a sequencing read according to the following conditions: a. removing a read with the C base and the G base both having a conversion frequency of less than 0.5; b. removing a read with the C base and the G base both having a conversion frequency of greater than 0.9; c. removing a sequence deemed to be erroneous; d. retaining a sequencing read with a conversion frequency of the C base less than 0.01 while a conversion frequency of the G base greater than 0.99; e. retaining a sequencing read with a conversion frequency of the G base less than 0.01 while a conversion frequency of the C base greater than 0.99; and f. obtaining a methylation haplotype; iii) extracting a haplotype with a sequencing depth greater than 10; and iiii) filtering out a haplotype with a methylation frequency of the C base being null.

In some specific embodiments, in the method for filtering the methylation sequencing data according to the present disclosure, the methylation haplotype may be obtained after reading and analyzing the methylation dependence between adjacent CpGs by the biscuit sliding window method.

In some specific embodiments, the methylation haplotypes that have completed the filtering of the methylation sequencing data are subjected to the Fisher's test. The present disclosure selects the differential methylation haplotypes specific to different subtypes of cancer cells that satisfy the following conditions: a significance indicated as p of the pairwise difference between the different subtypes of cancer cells and normal leukocytes is less than 0.05, a difference comparison of the methylation haplotypes is performed in a sample at a cancer cell DNA concentration of 1, each of the ratios among methylation levels of the different subtypes of cancer cells is required to be greater than 2, or greater than 5 or greater than 10, or less than 1/2, or less than 1/5 or less than 1/10, and furthermore, each of the ratios between methylation levels of the different subtypes of cancer cells and that of a normal leukocyte sample is required to be greater than 2, or greater than 5 or greater than 10, or less than 1/2, or less than 1/5 or less than 1/10.

In some specific embodiments, the methylation haplotypes that have completed the filtering of the methylation sequencing data are subjected to the Fisher's test. The present disclosure selects the differential methylation haplotypes specific to different subtypes of cancer cells that satisfy the following conditions: a significance indicated as p of the pairwise difference between different subtypes of cancer cells and normal leukocytes is less than 0.05, a difference comparison of the methylation haplotypes is performed in a sample at a cancer cell DNA concentration of 1, each of the ratios among methylation levels of the different subtypes of cancer cells is required to be greater than 2 or less than 1/2, and furthermore, each of the ratios between methylation levels of the different subtypes of cancer cells and that of a normal leukocyte sample is required to be greater than 2 or less than 1/2.

To be clearer, the technical solutions of the present disclosure are further illustrated below with reference to the examples, but they are not intended to limit the present disclosure, and instead they are only some examples of the present disclosure. Unless otherwise specified, the instruments, reagents, materials, experimental animals, etc. used herein are all commercially available.

### Example 1

### Experimental Materials

The experiment involved a total of 142 samples, of which there were a total of 84 samples used for training the models, including 33 tissue samples, 24 muscle invasive bladder urothelial carcinoma samples, and 9 non-muscle invasive bladder urothelial carcinoma samples; and there were 51 normal urine samples.

There were 58 preoperative urine samples for determining the model effects, of which there were 39 samples from patients with the muscle invasive bladder urothelial carcinoma and 19 samples from patients with the non-muscle invasive bladder urothelial carcinoma.

### Experiment

### 1. DNA Isolation

gDNA Isolation Kit purchased from Magen (D6315-03) or QIAGEN (69506) and operated according to the kit instructions.

cfDNA Isolation Kit purchased from Zymo (D3061) or Thermo Fisher (A29319) and operated according to the kit instructions.

### 2. DNA Bisulfite Conversion

The DNA bisulfite conversion kits were purchased from Zymo and operated according to the kit instructions. The conversion products were eluted with NF-H₂O for subsequent use.

### 3. Multiplex PCR Amplification

The multiplex PCR reagents were purchased from Thermo Fisher (N8080241).

The multiplex PCR primers were synthesized by Sangon Biotech (Shanghai) Co., Ltd. Specific operations:

**Table 1 Multiplex PCR Reaction System**

| **Reagents** | **Volume (µL)** |
|---|---|
| DNA (20 ng, initial amount) | X |
| 10× Buffer | 2 |
| 10 mM dNTPs | 0.4 |
| 25 mM MgCl₂ | 1.2 |
| AmplitaqGoldtaq | 0.1 |
| Primer | 4.6 |
| NF-H₂O | 11.7-X |
| Total | 20 |

PCR reaction procedures: 95°C for 10 min; 25 × (95°C for 30 s, 65°C for 30 s, 54°C for 2 min, 65°C for 30 s, 72°C for 30 s); 72°C for 10 min; stored at 16°C.

### 4. Recovery of Multiplex PCR Product

The amplified products were enriched and recovered using 24 µL of AMPure XP magnetic beads (Beckman, A63880) according to the instructions, and dissolved in 23 µL of sterilized water.

### 5. Index PCR

Reagents for index PCR were purchased from KAPA Biosystems (KK2602).

Specific operations:

**Table 2 Index PCR Reaction System**

| **Reagents** | **Volume (µL)** |
|---|---|
| Previously recovered product | 23 |
| 2 × PCR mix | 25 |
| i5 index | 1 |
| i7 index | 1 |
| Total | 50 |

PCR reaction procedures: 98°C for 45 s; 13 × (98°C for 15 s, 60°C for 30 s, 72°C for 30 s); 72°C for 5 min; stored at 4°C.

### 6. Recovery and Quantification of Index PCR Product

The amplified products were enriched and recovered using 60 µL of AMPure XP magnetic beads (Beckman, A63880) according to the instructions, and dissolved in 35 µL of sterilized water. The double-stranded quantification was performed using dsDNA Qubit.

### 7. High-Throughput Sequencing

After the concentrations of the libraries were assayed by fluorescence quantitative PCR and qualified, the libraries were subjected to the PE150 sequencing on the Novaseq platform of Illumina and the data volume for each library were 5M to 8M reads.

### 8. Data Analysis

### Haplotype Extraction

In the treatment method for DNA methylation sequencing, the DNA fragments were treated with bisulfite, and thereafter the unmethylated C bases would be converted into the T bases with the aid of a reduction reaction, while the methylated C bases remained unchanged. According to conventional analysis, the following two errors might possibly occur here: (1) the unmethylated C base has not been successfully converted into the T base after treatment; and (2) the methylated C base that should have remained unchanged has changed. Based on the common knowledge of the biology, it could be determined that the vast majority of C base methylations were all at the CpG sites, whereas the probability of methylation at the non-CpG sites was extremely low (less than 0.5%), so most of the methylated C bases should be at the CpG sites. Therefore, the "conversion efficiency" by methylation could be obtained by counting the proportion of the C bases converted into the T bases at the non-CpG sites. In case of correct experimental and analytical processes, the conversion efficiency by the bisulfite treatment method was very high and could reach a level higher than 99%.

The present inventors had unexpectedly discovered that surprisingly, the conversion rates at the non-CpG sites of the same sequencing read were always correlated with one another. This phenomenon was actually not mysterious because the sulfite conversion required the double strands to be unwound into a single-stranded structure. It could be concluded that the higher the conversion rate of the C bases at the non-CpG sites on one sequencing read, the higher the accuracy of the methylation treatment. If there was any one unconverted C base at the non-CpG site, this indicated a high probability for other C bases that were still not converted, and this class of sequences had low credibility. As a result, filtering this class of sequences could further improve the accuracy and credibility of methylation analysis. First of all, the CpG sites were eliminated from the corresponding reference sequences and the remaining sequences are the ones containing only the non-CpG base sites, then the conversion rates at these sites were calculated, and finally the desired data were obtained by filtering according to the quality control conditions.

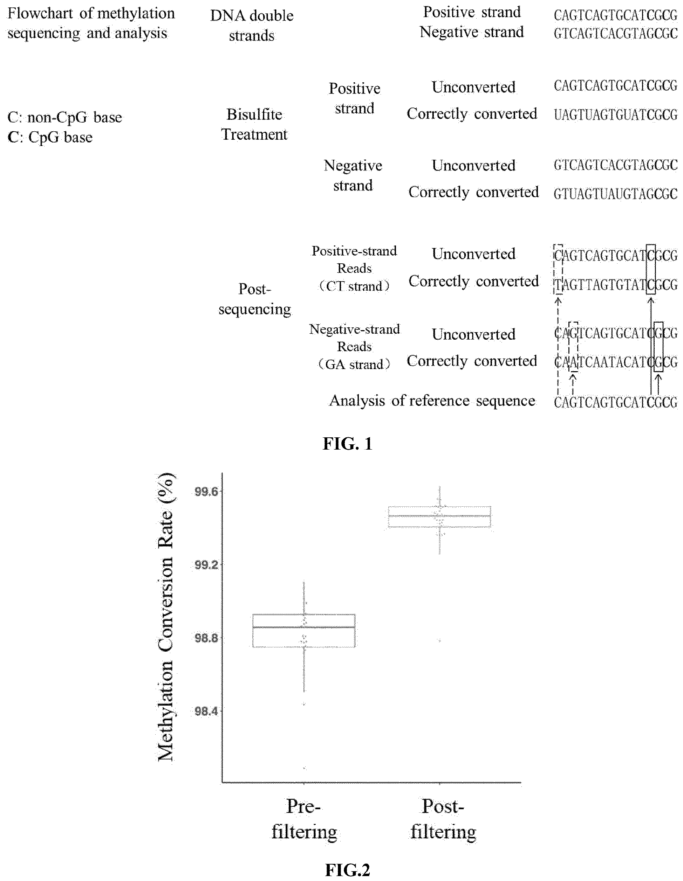

Specifically, the present disclosure calculated the conversion rate by the following method: counting the number of unconverted bases on the sequencing reads based on the reference sequence (based on the principle of complementary pairing of the double strands of DNA, counting the number of the C bases on the positive-strand sequencing reads, *i.e.,* positive-strand reads, and counting the number of the G bases on the negative-strand sequencing reads, *i.e.,* negative-strand reads, referring to FIG. 1 for the principle), and dividing the counted number of the C bases or the G bases by the total number of the bases at its respective corresponding site to obtain the non-conversion rates of the C bases and the G bases respectively, while the corresponding conversion rate was equal to 1 - the non-conversion rate.

Specifically, the present disclosure filters the sequencing reads according to the following conditions: ① removing the reads with the C base and the G base both having a conversion frequency of <0.5, optionally <0.6, <0.7, <0.8 or <0.9; ② removing the reads with the C base and the G base both having a conversion frequency of >0.9, optionally >0.8, >0.7, >0.6 or >0.5; and removing the sequences deemed to be erroneous, which included, but were not limited to, an amplification error, a sequencing error, an alignment error, etc.; ③ retaining the sequencing reads with a low conversion rate of the C bases while a high conversion rate of the G bases, optionally the conversion rate of the C bases being <0.2, <0.1 or <0.01, while the high conversion rate of the G bases being >0.99, >0.9 or >0.8; and ④ retaining the sequencing reads with a low conversion rate of the G bases while a high conversion rate of the C bases, optionally the conversion rate of the G bases being <0.2, <0.1 or <0.01, while the conversion rate of the C bases being >0.99, >0.9 or >0.8.

Specifically, for the data obtained after filtering, the present disclosure read and analyzed the methylation dependence between the adjacent CpGs by the biscuit sliding window method to obtain the methylation haplotypes, which were stored in the epiread format, and then worked out the methylation levels of the haplotypes for testing.

Specifically, the 183 target samples for test were analyzed and the statistical results before and after filtering showed that the screening rate of low-quality DNA fragments was 0.8% to 3% and the average screening rate was 2.05%. Furthermore, as shown in FIG. 2, the conversion rate at the non-CpG sites was increased from 98.81% to 99.44% after filtering, which demonstrated that the filtered sequences carried about 63% of unconverted C bases. By filtering out the incompletely converted DNA fragments, the present disclosure effectively improved the accuracy.

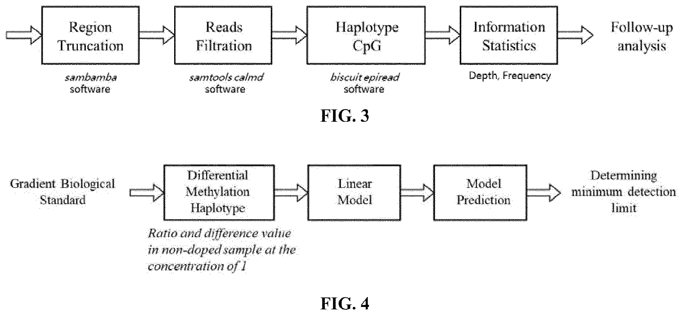

The sequencing sequence in the insertion region during primer synthesis was truncated, which was the truly valid sequence with qualified depth that exactly fell in the designed primer region. The number of sequences with a different haplotype in the region was calculated as the sequencing depth of this haplotype, and the frequency of this haplotype in the region was also calculated. The haplotypes with the depth of N>10 were eventually extracted, and the haplotypes with the C base frequency (freqC) of null were eliminated because they were sequences with alignment errors. Finally, the haplotypes were distinguished by the naming method of the primer region + the methylation freqC, which were the results of haplotypes with reliable quality and high accuracy and credibility. The process was as shown in FIG. 3.

### Example 2

Bladder urothelial carcinoma cell lines RT4 (non-muscle invasive NMIBC type) and 5637 (muscle invasive MIBC type) as well as normal human leukocytes were used for testing biological standards to specify the minimum content detection limits for the bladder urothelial carcinoma and its subtypes in the present disclosure.

The specific process was as follows:

### 1. gDNA Isolation

The bladder urothelial carcinoma cell lines and the normal human leukocytes were purchased from the Cell Bank of the Chinese Academy of Sciences. The isolation kits were purchased from QIAGEN (69506) and operated according to the kit instructions.

### 2. DNA Bisulfite Conversion (referring to Example 1)

### 3. Gradient Doping of Biological Standards

After bisulfite conversion, the RT4/5637/leukocytes gDNAs were ultrasonically sheared to stimulate cfDNAs. The normal urine cfDNAs were subjected to single-stranded quantification without ultrasonic shearing. Gradient doping was performed according to the DNA concentration. A total of 8 pattern standard gradients were set for each group in the present application (*i.e.,* the bladder urothelial carcinoma DNA contents were 100%, 20%, 10%, 5%, 2%, 1%, 0.5%, and 0%).

### 4. Methylation multiplex PCR, index PCR and high-throughput sequencing (referring to Example 1)

### 5. Data Analysis. The method for haplotype extraction was as described in Example 1.

Each of the haplotypes was statistically tested by the Fisher's test and required to satisfy that: the significance of pairwise difference between the muscle invasive bladder urothelial carcinoma as well as the non-muscle invasive bladder urothelial carcinoma and the normal leukocytes was p<0.05. Afterwards, the difference comparison of the methylation haplotypes was carried out in samples of pure tissue (*i.e.,* the tumor DNA content was 1), requiring that the ratio between the methylation levels of the muscle invasive and non-muscle invasive bladder urothelial carcinoma samples was greater than 2 or less than 1/2 (including, but not limited to >5 or >10, or <1/5 or <1/10) and further requiring that both the ratios between the methylation levels of the two species of bladder urothelial carcinoma samples and the normal leukocyte samples satisfied the condition of greater than 2 or less than 1/2 (including, but not limited to >5 or >10, or <1/5 or <1/10). Based on the above standard, a total of 60 cancer-/tumor-specific haplotypes were picked out (including 32 muscle invasive haplotypes and 28 non-muscle invasive haplotypes). Additionally, the multiples of the differences between methylation level of the leukocyte sample and those of the two species of carcinoma samples were required to be greater than 3 and the haplotype frequencies of the carcinoma samples were not less than 0.0001, by which 31 leukocyte-specific haplotypes were picked out. The process was as shown in FIG. 4.

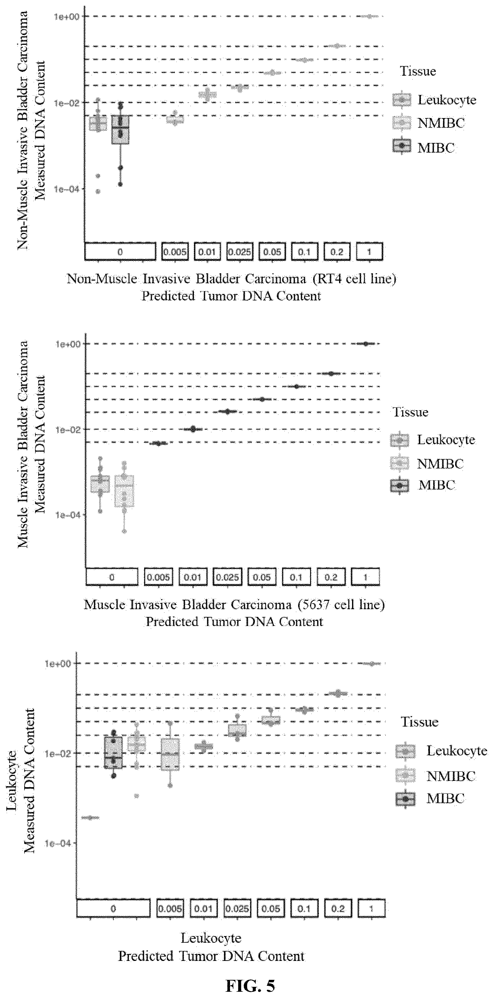

Three categories of linear models were created. The leukocyte-specific haplotype + the muscle invasive haplotype or the non-muscle invasive haplotype were fitted respectively to create two models; and furthermore, the leukocyte-specific haplotype alone was used to create the third model, so as to predict the methylation levels of the samples with different concentration gradients and compare them with the actual doping concentrations. As shown in FIG. 5, the minimum detection limits of the MIBC and NMIBC models were at 0.005, and the minimum detection limit of the leukocyte-specific model was at 0.01.

### Example 4

The data analysis method of the present application was applied to the detection for bladder urothelial carcinoma.

The sequencing data on the clinical samples in Example 1 were employed.

The specific analysis process was as follows:

### Model and Method

### (1) Difference in source of urine

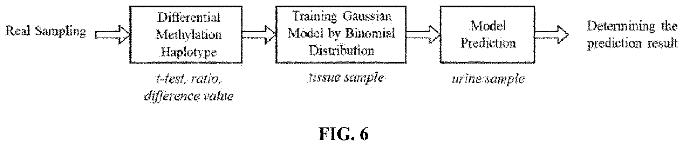

The authentic tissue samples involved muscle invasive samples and non-muscle invasive samples. The t-test models were used to test the differences between the two species of cancer samples and the normal urine samples as well as the difference between the methylation levels of the two species of cancer samples. Haplotypes with the significance of difference p<0.05 in the three test results were selected, and the categories of haplotypes were distinguished depending upon whether the logarithm of the mean frequency difference is positive or negative. As a result, a total of 42 haplotypes were picked out, including four categories, *i.e.,* 10 haplotypes with lower frequency in the muscle invasive samples than in the non-muscle invasive samples (m_n_negative), 20 haplotypes with higher frequency in the muscle invasive samples than in the non-muscle invasive samples (m_n_positive), 8 haplotypes with higher frequency in the cancer samples than in the normal urine samples (u_negative),and 4 haplotypes with lower frequency in the cancer samples than in the normal urine samples(u_positive), respectively. The models were trained in tissue samples and used to make predictions in the tissue and urine samples, respectively. The process was as shown in FIG. 6.

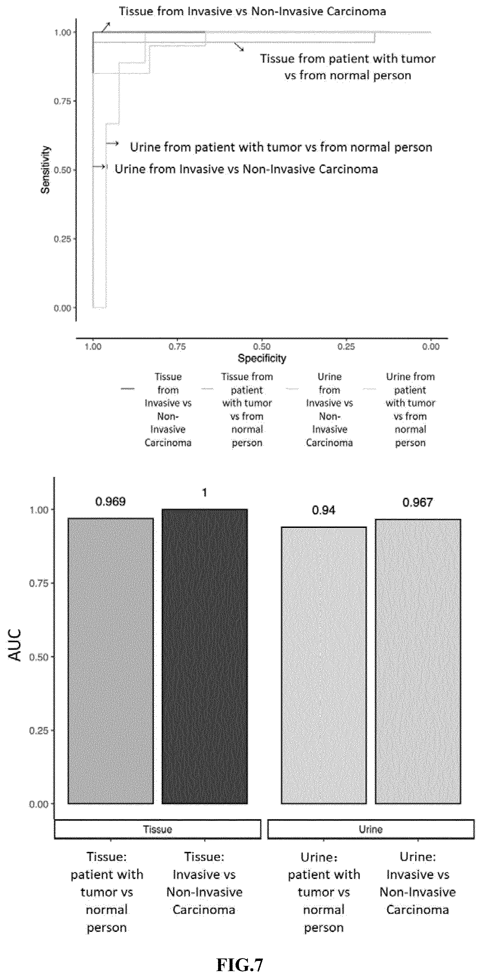

The prediction results were as shown in FIG. 7. The haplotypes filtered by the present method could well distinguish the bladder urothelial carcinoma tissue and the normal urine (AUC=1.0), and had a better degree of distinction between the muscle invasive and non-muscle invasive samples in the bladder urothelial carcinoma tissue (AUC=0.969). In addition, in all the urine samples, the present inventors could also find a good degree of distinction between the urine from patients with the muscle invasive and non-muscle invasive bladder urothelial carcinoma (AUC=0.967), and a good degree of distinction between the urine from patients with the bladder urothelial carcinoma and the urine from a normal person (AUC=0.94).

For urine cfDNA, the present inventors worked out the sensitivity and specificity of the urine samples based on the data. In the present application, when a patient with bladder urothelial carcinoma and a normal person were diagnosed using urine, the sensitivity was 0.889 and the specificity was 0.945; when the subtyping muscle invasive bladder urothelial carcinoma and the subtyping non-muscle invasive bladder urothelial carcinoma were identified using urine, the sensitivity was 0.850 and the specificity was 1.000.

### Example 5

### Experimental Materials

The experiment involved a total of 142 samples, and the specific samples were as same as those in Example 1.

### Experiment

The experiment included ① DNA isolation; ② DNA bisulfite conversion; ③ multiplex PCR amplification and product recovery; ④ index PCR and product recovery; ⑤ high-throughput sequencing; and ⑥ data analysis.

The specific operating steps in ① to ⑤ were as same as those in Example 1.

The operating steps in ⑥ were as follows:
(1) the obtained methylation sequencing data were input in the apparatus for filtering the methylation sequencing data;
(2) the processor was operated to execute the instructions stored in the memory and implement the method for filtering the methylation sequencing data, the method comprising the following steps: i) truncating a sequencing sequence on an insertion region during primer synthesis; ii) filtering a sequencing read according to the following conditions to obtain a methylation haplotype of a satisfactory sequencing read: ① removing the reads with the C bases and the G bases both having a conversion frequency of <0.5, optionally <0.6, <0.7, <0.8 or <0.9; ② removing the reads with the C bases and the G bases both having a conversion frequency of >0.9, optionally >0.8, >0.7, >0.6 or >0.5; ③ removing the sequences deemed to be erroneous including, but not limited to an amplification error, a sequencing error, an alignment error, etc.; ④ retaining the sequencing reads with a low conversion rate of the C bases while a high conversion rate of the G bases, optionally a conversion rate of the C bases of <0.2, <0.1 or <0.01, while a conversion rate of the G bases of >0.99, >0.9 or >0.8; ⑤ retaining the sequencing reads with a low conversion rate of the G bases while a high conversion rate of the C bases, optionally a conversion rate of the G bases of <0.2, <0.1 or <0.01, while a conversion rate of the C bases of >0.99, >0.9 or >0.8; iii) reading and analyzing the methylation dependence between adjacent CpGs by the biscuit sliding window method to obtain the methylation haplotype, storing the methylation haplotype in the epiread format, calculating the methylation level of each of the haplotypes, and extracting the haplotypes with a sequencing depth greater than 10; and iiii) filtering out the haplotypes with a methylation frequency of the C bases being null;
(3) the remaining sequencing reads were output to obtain the methylation haplotypes;
(4) the respective methylation haplotypes were distinguished by the naming method of primer region + methylation freqC, which were the results of the finally obtained haplotypes with reliable quality and high accuracy and credibility.

After the above treatment, the conversion rate at the non-CpG sites was increased from 98.81% to 99.44%.

### Example 6

### Experimental Materials

The experiment involved a total of 142 samples and biological standards. The specific samples were as same as those in Example 1 and the biological standards were as same as those in Example 2.

### Experiment

The experiment included ① DNA isolation; ② DNA bisulfite conversion; ③ gradient doping of biological standards; ④ multiplex PCR amplification and product recovery; ⑤ index PCR and product recovery; ⑥ high-throughput sequencing; and ⑦ data analysis.

The specific operating steps in ① to ⑥ were as same as those in Example 2.

The operating steps in ⑦ were as follows:
(1) the obtained methylation sequencing data were input in an apparatus for selecting a DNA methylation haplotype capable of determining whether a subject suffered from cancer or subtyping a patient with cancer;
(2) the processor was operated to execute the instructions stored in the memory and implement the method for selecting the DNA methylation haplotype capable of determining whether the subject suffers from cancer or subtyping the patient with cancer, the method generally comprising three major steps:
   i) a step of filtering methylation sequencing data: a. truncating a sequencing sequence on an insertion region during primer synthesis; b. filtering a sequencing read according to the following conditions to obtain a methylation haplotype of a satisfactory sequencing read: removing a read with the C base and the G base both having a conversion frequency of <0.5, optionally <0.6, <0.7, <0.8 or <0.9; removing a read with the C base and the G base both having a conversion frequency of >0.9, optionally >0.8, >0.7, >0.6 or >0.5; removing a sequence deemed to be erroneous including, but not limited to an amplification error, a sequencing error, an alignment error, etc.; retaining a sequencing read with a low conversion rate of the C base while a high conversion rate of the G base, optionally a conversion rate of the C base of <0.2, <0.1 or <0.01, while a conversion rate of the G base of >0.99, >0.9 or >0.8; retaining a sequencing read with a low conversion rate of the G base while a high conversion rate of the C base, optionally a conversion rate of the G base of <0.2, <0.1 or <0.01, while a conversion rate of the C base of >0.99, >0.9 or >0.8; c. reading and analyzing the methylation dependence between adjacent CpGs by the biscuit sliding window method to obtain the methylation haplotype, storing the methylation haplotype in the epiread form, calculating the methylation level of each of the haplotypes, and extracting a haplotype with a sequencing depth greater than 10; d. filtering out a haplotype with a methylation frequency of the C base being null; and e. outputting the remaining sequencing sequences to obtain the methylation haplotypes;
   ii) a step of selecting a differential methylation haplotype: a. performing a Fisher's test on the obtained methylation haplotype, and selecting the differential methylation haplotype specific to different subtypes of cancer cells that satisfied the following conditions: significances indicated as p of pairwise difference between the muscle invasive and non-muscle invasive bladder urothelial carcinoma cancer cells and the normal leukocytes were less than 0.05, a difference comparison of the methylation haplotypes was performed in a sample with a cancer cell DNA concentration of 1, requiring that the ratio between the methylation levels of different subtypes of cancer cells was greater than 2 or less than 1/2 (including, but not limited to >5 or >10, or <1/5 or <1/10), and furthermore requiring that both the ratios between the methylation levels of the muscle invasive and non-muscle invasive bladder urothelial carcinoma samples and the normal leukocyte samples were greater than 2 or less than 1/2 (including, but not limited to >5 or >10, or <1/5 or <1/10); selecting a differential methylation haplotype specific to leukocytes that satisfied the following condition: the multiples of the differences between methylation level in aleukocyte and those in the two categories of cancer samples were greater than 3 and the haplotype frequencies in the cancer samples were not less than 0.0001; and thus obtaining the selected differential methylation haplotype; b. creating the following linear models and obtaining the minimum detection limit of the cancer cell DNA content: a linear model with the abundance of the differential methylation haplotype specific to a normal human leukocyte and the abundance of the differential methylation haplotype specific to a muscle invasive bladder urothelial carcinoma cancer cell varying with the cancer cell DNA concertation; a linear model with the abundance of the differential methylation haplotype specific to a normal human leukocyte and the abundance of the differential methylation haplotype specific to a non-muscle invasive bladder urothelial carcinoma cancer cell varying with the cancer cell DNA concertation; and a linear model with the abundance of the differential methylation haplotype specific to the normal human leukocyte varying with the normal leukocyte DNA concentration; and
   iii) a step of training the differential methylation haplotype: a. for the selected differential methylation haplotypes, detecting the differences between the methylation levels of the muscle invasive and non-muscle invasive bladder urothelial carcinoma cancer tissue samples and the normal urine sample and the difference between the methylation levels of the two cancer samples, selecting a haplotype with a significance indicated as p of difference less than 0.05 in three t-test results, and categorizing the haplotypes depending upon whether a logarithm of a mean difference value of methylation level was positive or negative, and accordingly making a determination in a tissue sample and a urine sample respectively, calculating the degrees of distinction of the selected differential methylation haplotype to the bladder urothelial carcinoma sample and the normal human sample as well as to the muscle invasive bladder urothelial carcinoma sample and the non-muscle invasive bladder urothelial carcinoma sample, and the sensitivity and specificity of the selected differential methylation haplotype;
(3) the obtained results were output: a. the methylation haplotypes obtained by screening in step i); b. the selected differential methylation haplotypes in step ii); c. the minimum detection limit of each of the models obtained in step ii); d. the differential methylation haplotypes that had passed the methylation level test and the categories thereof in step iii); and e. the degrees of distinction (AUC) of the differential methylation haplotypes to the samples as well as the sensitivity and specificity thereof obtained by tests in step iii).

After the above treatment, the conversion rate at the non-CpG site was increased from 98.81% to 99.44%. The following methylation haplotypes were eventually selected: 10 methylation haplotypes with lower haplotype frequency in the muscle invasive bladder urothelial carcinoma than in the non-muscle invasive bladder urothelial carcinoma; 20 methylation haplotypes with higher haplotype frequency in the muscle invasive bladder urothelial carcinoma than in the non-muscle invasive bladder urothelial carcinoma; 8 haplotypes with higher haplotype frequency in the cancer samples than in the normal urine samples; and 4 haplotypes with higher haplotype frequency in the cancer samples than in the normal urine samples. These haplotypes had AUC of 1.0 for distinguishing the bladder urothelial carcinoma tissue from the normal urine, AUC of 0.969 for distinguishing the muscle invasive bladder urothelial carcinoma tissue from the non-muscle invasive bladder urothelial carcinoma tissue, AUC of 0.967 for distinguishing the urine of the patient with the muscle invasive bladder urothelial carcinoma from the urine of the patient with the non-muscle invasive bladder urothelial carcinoma, and AUC of 0.94 for distinguishing the urine of the patient with the bladder urothelial carcinoma from the urine of the normal person. In the present application, when the bladder urothelial carcinoma patient and the normal person were diagnosed using urine, the sensitivity was 0.889 and the specificity was 0.945; and when the subtyping muscle invasive bladder urothelial carcinoma and the subtyping non-muscle invasive bladder urothelial carcinoma were identified using urine, the sensitivity was 0.850 and the specificity was 1.000.

## Claims

1. An apparatus for filtering methylation sequencing data, comprising:
a processor; and
a memory configured to store executable instructions;
wherein the processor, when executing instructions, implements a method for filtering the methylation sequencing data, the method comprising the following steps:
i) truncating a sequencing sequence on an insertion region during primer synthesis;
ii) filtering a sequencing read according to the following conditions to obtain a methylation haplotype of the sequencing read that satisfies the conditions:
a. removing a read with a C base and a G base both having a conversion frequency of less than 0.9; b. removing a read with a C base and a G base both having a conversion frequency of greater than 0.5; c. removing a sequence deemed to be erroneous; d. retaining a sequencing read with a conversion frequency of a C base less than 0.2 while a conversion frequency of a G base greater than 0.8; and e. retaining a sequencing read with a conversion frequency of a G base less than 0.2 while a conversion frequency of a C base greater than 0.8;
iii) extracting a haplotype with a sequencing depth greater than 10; and
iiii) filtering out a haplotype with a methylation frequency of a C base being null.

2. The apparatus according to claim 1, wherein the methylation sequencing data is obtained by bisulfite sequencing.

3. An apparatus for selecting a DNA methylation haplotype capable of determining whether a subject suffers from cancer or determining subtypes of cancer in a patient, comprising:
a processor; and
a memory configured to store executable instructions;
wherein the processor, when executing instructions, implements a method for selecting the DNA methylation haplotype capable of determining whether a subject suffers from cancer or determining subtypes of cancer in a patient, the method comprising the following steps:
a step of filtering methylation sequencing data;
a step of selecting differential methylation haplotypes; and
a step of training the differential methylation haplotypes.

4. The apparatus according to claim 3, wherein the step of filtering methylation sequencing data comprises:
i) truncating a sequencing sequence on an insertion region during primer synthesis;
ii) filtering a sequencing read according to the following conditions to obtain a methylation haplotype of the sequencing read that satisfies the conditions: a. removing a read with a C base and a G base both having a conversion frequency of less than 0.9; b. removing a read with a C base and a G base both having a conversion frequency of greater than 0.5; c. removing a sequence deemed to be erroneous; d. retaining a sequencing read with a conversion frequency of a C base less than 0.2 while a conversion frequency of a G base greater than 0.8; and e. retaining a sequencing read with a conversion frequency of a G base less than 0.2 while a conversion frequency of a C base greater than 0.8;
iii) extracting a haplotype with a sequencing depth greater than 10; and
iiii) filtering out a haplotype with a methylation frequency of a C base being null.

5. The apparatus according to claim 3, wherein the step of selecting differential methylation haplotypes comprises:
performing a Fisher's test on the methylation haplotypes that have completed the step of filtering methylation sequencing data, and selecting differential methylation haplotypes specific to different subtypes of cancer cells that satisfy the following conditions:
a significance indicated as p of pairwise difference between the different subtypes of cancer cells and normal leukocytes is less than 0.05, a difference comparison of the methylation haplotype is performed in a sample at a cancer cell DNA concentration of 1, each of the ratios among methylation levels of the different subtypes of cancer cells is required to be greater than 2 or less than 1/2, and each of the ratios between methylation levels of the different subtypes of cancer cells and that of a normal leukocyte sample is additionally required to be greater than 2 or less than 1/2; the differential methylation haplotype specific to leukocytes that satisfies the following condition is selected: multiples of differences between methylation level of a leukocyte sample and those of the different subtypes of cancer samples are greater than 3, and a haplotype frequency in the cancer samples is not less than 0.0001;
creating the following linear models and obtaining a minimum detection limit of a cancer cell DNA content:
i) the linear models with the respective sums of an abundance of the differential methylation haplotype specific to a normal human leukocyte and an abundance of each of the differential methylation haplotypes specific to the different subtypes of cancer cells varying with a cancer cell DNA concertation; and
ii) the linear model with an abundance of the differential methylation haplotype specific to a normal human leukocyte varying with a normal leukocyte DNA concentration as variables.

6. The apparatus according to claim 3, wherein the step of training the differential methylation haplotypes comprises:
detecting differences between methylation levels of different subtypes of cancer tissue samples and that of a normal urine sample and the difference among the methylation levels of the different subtypes of cancer samples, selecting a haplotype with a significance indicated as p of difference less than 0.05 in three t-test results, and categorizing the haplotype depending upon whether a logarithm of a mean difference value of the methylation levels is positive or negative, and accordingly making a determination in a tissue sample and a urine sample respectively, and calculating sensitivity and specificity of a selected differential methylation haplotype for distinguishing the cancer samples from a normal human sample and for determining the different subtypes of the cancer samples.

7. The apparatus according to any one of claims 3 to 6, wherein the methylation sequencing data is obtained by bisulfite sequencing.

8. The apparatus according to any one of claims 3 to 6, wherein the sample is gDNA or cfDNA.

9. The apparatus according to any one of claims 3 to 6, wherein the cancer is bladder urothelial carcinoma.

10. The apparatus according to any one of claims 3 to 6, wherein the subtypes of cancer are muscle invasive bladder urothelial carcinoma and non-muscle invasive bladder urothelial carcinoma.
